(19) Europäisches Patentamt
European Patent Office
Office européen des brevets

(11) EP 0 845 257 B1

(12) **FASCICULE DE BREVET EUROPEEN**

(45) Date de publication et mention de la délivrance du brevet:
**14.01.2004 Bulletin 2004/03**

(51) Int Cl.$^7$: **A61K 7/06**, A61K 7/00

(21) Numéro de dépôt: **97402755.9**

(22) Date de dépôt: **17.11.1997**

(54) **Dispositif aérosol à base de compositions alcooliques de matériaux fixants comprenant des motifs vinyl lactames**

Aerosolvorrichtung auf der Basis von alkoholischen Zusammensetzungen festigenderStoffe, die Vinyllaktam-Einheiten enthalten

Aerosol device based on alcoholic compositions of binding materials containing vinyl-lactam units

(84) Etats contractants désignés:
**AT BE CH DE DK ES FI FR GB GR IE IT LI LU MC NL PT SE**

(30) Priorité: **22.11.1996 FR 9614330**

(43) Date de publication de la demande:
**03.06.1998 Bulletin 1998/23**

(73) Titulaire: **L'OREAL**
**75008 Paris (FR)**

(72) Inventeurs:
- **Samain, Henri**
  **91570 Bievres (FR)**
- **Dupuis, Christine**
  **75018 Paris (FR)**

(74) Mandataire: **Andral, Christophe André Louis**
**L'OREAL - D.I.P.I.**
**25-29 Quai Aulagnier**
**92600 Asnières (FR)**

(56) Documents cités:
**WO-A-95/00105**
**WO-A-95/33437**
**WO-A-96/06592**
**DE-A- 4 414 423**
**DE-A- 4 414 424**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 845 257 B1

Printed by Jouve, 75001 PARIS (FR)

## Description

La présente invention concerne de nouveaux dispositifs aérosols destinés à fixer les chevelures.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

On connaît de nombreux systèmes aérosols destinés à fixer les chevelures, ces systèmes contenant d'une part une phase liquide (ou jus) et d'autre part un propulseur. Le jus contient les matériaux fixants et un solvant approprié. Le propulseur a pour fonction d'assurer une pression permettant la pulvérisation de la phase liquide, et son application sur les cheveux sous forme d'un nuage de gouttelettes dispersées. C'est une fois appliquée sur les cheveux que la phase liquide sèche, permettant la formation de soudures nécessaires à la fixation de la chevelure par les matériaux fixants.

Les soudures doivent être suffisamment rigides pour assurer le maintien des cheveux. Cependant, elles doivent également être suffisamment fragiles pour que l'utilisateur puisse, en peignant ou brossant la chevelure, les détruire sans heurter le cuir chevelu ni endommager les cheveux.

Les matériaux fixants sont généralement des polymères fixants, c'est à dire des polymères filmogènes solubles ou dispersibles dans l'eau et dans l'alcool, tels que par exemple les copolymères d'acétate de vinyle/acide crotonique, les résines acryliques anioniques ou amphotères. Ces matériaux permettent d'obtenir facilement l'effet fixant, en revanche, après brossage ou peignage, les cheveux présentent dans les conditions usuelles de laquage un aspect raidi et un toucher rêche, voire collant, et un démêlage souvent difficile.

Ces inconvénients sont liés à plusieurs paramètres, parmi lesquels on peut citer la nature du ou des polymères fixants, ou encore à la nature des soudures. Pour remédier à ces inconvénients on peut donc agir sur ces deux paramètres, sans toutefois diminuer l'effet fixant recherché. Pour améliorer les propriétés cosmétiques des matériaux fixants, il a surtout été proposé d'associer différents polymères (WO 94/12148, WO 96106592, US 5 158 762).

La Demanderesse a maintenant trouvé qu'en sélectionnant de manière appropriée d'une part les polymères fixants et d'autre part les paramètres de diffusion des compositions, il était possible d'agir sur la qualité des soudures tout en conservant de bonnes qualités de fixation et/ou de mise en forme la coiffure, et donc apporter d'excellentes propriétés cosmétiques telles que la douceur, le démêlage.

Le dispositif aérosol selon l'invention est constitué par un récipient contenant une composition aérosol constituée d'une part par une phase liquide (ou jus) contenant au moins un matériau fixant dans un solvant approprié et d'autre part un propulseur, et un moyen de distribution de ladite composition aérosol, caractérisé en ce que le matériau fixant a une température de transition vitreuse (Tg) supérieure ou égale à 50 °C, ledit matériau fixant comprenant au moins un polymère à motifs vinyl lactames, et en ce que le dispositif est approprié pour obtenir un pouvoir mouillant supérieur ou égal à 50 mg/s et possède une valve droite avec un gicleur de diamètre compris entre 0,35 et 0,60 mm, le matériau fixant étant essentiellement constitué par le polymère à motifs vinyl lactames.

Le moyen de distribution est généralement constitué par une valve de distribution commandée par une tête de distribution, elle même comprenant une buse par laquelle la composition aérosol est vaporisée.

Les notions de « Tg » et « pouvoir mouillant » telles qu'on les entend selon la présente invention sont définies ci-dessous.

Par température de transition vitreuse (Tg), on entend selon la présente invention la Tg du matériau fixant dans l'extrait sec, l'extrait sec étant constitué par l'ensemble des matériaux non volatiles dans le jus, ou matière sèche.

Selon la présente invention, le pouvoir mouillant correspond à la quantité de produit reçue par une feuille de matière plastique disposée à une distance de 35 cm de la buse du dispositif aérosol pendant une unité de temps donnée. Le produit est alors constitué par la matière sèche, plus une partie du solvant non évaporé en cours du trajet, plus éventuellement une partie du propulseur non évaporé. Ce pouvoir mouillant est exprimé en mg/s, et est mesuré selon l'invention par la méthode suivante:

- on suspend verticalement une feuille de matière plastique de dimensions 21 cm x 23 cm à une balance de précision (1/1000), la feuille étant reliée à la balance par le bord supérieur (généralement par un crochet de la balance inséré dans une perforation disposée au centre de la largeur et à 1 cm du bord supérieur de la feuille), et maintenue verticale par l'application d'un poids centré sur le bord inférieur (généralement par une pince fixée et centrée sur le bord inférieur);
- on place une cale derrière le bord inférieur de la feuille pour maintenir la feuille verticale lors de l'impact du produit;
- on dispose verticalement le dispositif aérosol de manière que la buse de diffusion de la composition soit disposée au centre et à 35 cm de la feuille verticale, pour une vaporisation du produit perpendiculaire à la feuille;

- on vaporise la composition pendant 5 secondes;
- on mesure la quantité de produit reçue sur la feuille verticale dès la fin de la vaporisation.

Pour plus de précision, on peut employer un dispositif approprié comprenant un moyen support du dispositif aérosol, et des moyens permettant le réglage tridimensionnel de la position de la buse par rapport à la feuille verticale. Ce dispositif peut être également équipé d'un dispositif pneumatique de contrôle du spray (déclenchement et durée), de manière à contrôler avec précision la durée de la vaporisation. L'ensemble peut être contrôlé par un ordinateur.
Pour éviter les perturbations environnementales, le trajet du produit entre la buse et la feuille sera avantageusement protégé horizontalement et verticalement par les parois d'un tunnel de dimensions appropriées.
Enfin, la vaporisation du produit est avantageusement effectuée sous atmosphère contrôlée, de manière préférentielle à une température de 20°C et une humidité relative de 50%.
De manière préférentielle. le matériau fixant a une Tg supérieure ou égale à 60 °C.
le matériau fixant est essentiellement constitué par le polymère à motifs vinyl lactames, seul ou en combinaison avec des additifs cosmétiques usuels, par exemple des plastifiants.
Selon l'invention, les polymères à motifs vinyl lactames sont ceux qui comprennent des motifs de formule

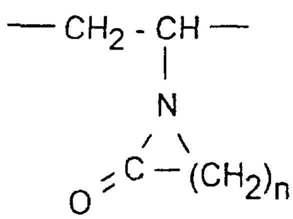

dans laquelle n est indépendamment 3, 4 ou 5.
De manière avantageuse, les polymères à motifs vinyl lactames sont des copolymères ou peuvent également comprendre des motifs de formule

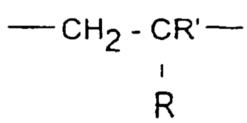

dans laquelle

R représente indépendamment un radical carboxy, un radical carbalcoxy, un radical acyloxy, le groupement alkoxy du radical carbalcoxy pouvant être substitué par au moins un radical hydroxy, amino, alkylamino ou dialkylamino, ou un radical aryle, en particulier phényle, éventuellement substitué par au moins un radical alkyle, et
R' représente un atome d'hydrogène ou un radical alkyle.

Par alkyle, on entend de préférence selon l'invention les radicaux alyles linéaires ou ramifiés en $C_1$-$C_{10}$, plus préférentiellement les radicaux alkyles en $C_1$-$C_4$, en particulier les radicaux méthyle, éthyle, n-propyle, i-propyle, n-butyle et t-butyle.
Par acyle, on entend de préférence selon l'invention les radicaux acyles dont le reste alkyle est un alkyle linéaire ou ramifié en $C_1$-$C_{10}$, en particulier les radicaux acétyle ou propionyle.
Les polymères à motifs vinyl lactames selon l'invention sont de manière préférentielle des polymères non ioniques ou faiblement cationiques. Ils sont notamment décrits dans les brevets US 3 770 683, US 3 929 735, US 4 521 504, US 5 158 762, US 5 506 315 et dans les demandes de brevet WO 941121148, WO 96/06592 et WO 96/10593. Ils peuvent se présenter sous forme pulvérulente ou sous forme de solution ou de suspension.
Parmi les polymères à motifs vinyl lactames utiles selon l'invention, on citera plus particulièrement les polyvinyl pyrrolidones, les polyvinyl caprolactames, les copolymères polyvinyl pyrrolidone / acétate de vinyle, les copolymères polyvinyl pyrrolidone / méthacrylate de diaminoéthyle non quaternisé (en particulier commercialisés sous les dénominations Copolymère 845, Copolymère 958 et Copolymère 937 par la société ISP), les terpolymères polyvinyl pyrrolidone / acétate de vinyle / propionate de vinyle (en particulier commercialisés sous la dénomination Luviskol VAP 343 par la société BASF), les terpolymères vinyl caprolactame / vinyl pyrrolidone / méthacrylate de diméthyl amino éthyle de Tg d'nviron 170 °C (en particulier commercialisé sous les dénominations Gaffix VC 713, $H_2$OLD, ACP 1187 et ACP

1189 par la société ISP) et les terpolymères acide (méth)acrylique / (méth)acrylates / vinyl pyrrolidone (en particulier commercialisés sous les dénominations Stepanhold Extra par la société STEPAN, Luvimer VBM 35 et Luvimer VBM 70 par la société BASF).

Les polymères à motifs vinyl lactames utiles selon l'invention sont de préférence des polymères à rigidification rapide. Par polymères à rigidification rapide, on entend selon l'invention des polymères qui conduisent à des soudures rigides en moins de 10 minutes.

La masse moléculaire en nombre du polymère à motifs vinyl lactames est généralement suppérieure à environ 5 000, de préférence comprise entre 10 000 et 1 000 000 environ, plus préférentiellement comprise entre 10 000 et 100 000 environ.

Le matériau fixant peut être utilisés sous forme solubilisée ou sous forme de dispersions de particules solides de polymère.

De manière avantageuse, le dispositif aérosol selon l'invention est approprié pour obtenir un pouvoir mouillant compris entre 50 mg/s et 500 mg/s.

Selon un mode préférentiel de réalisation de l'invention, le dispositif aérosol selon l'invention est approprié pour obtenir un débit en matière sèche supérieur ou égal à 20 mg/s, de préférence compris entre 20 mg/s et 60 mg/s.

Selon la présente invention, le débit en matière ($D_{MS}$) sèche correspond ne la quantité d'extrait sec qui sort du dispositif aérosol par unité de temps. Ce débit en matière sèche est exprimé en mg/s et est calculé en multipliant la concentration en matière sèche dans la composition aérosol ($C_{MS}$) par le débit de la composition aérosol à la sortie de la buse ($D_{CA}$):

$$D_{MS} = C_{MS} \text{ x } D_{CA}.$$

La concentration en matière sèche dans la composition aérosol ($C_{MS}$) correspond à la quantité de matière sèche ramenée à 100g de composition aérosol (jus + propulseur). La concentration en matière sèche est exprimée en pourcentage et est mesurée après pulvérisation par évaporation des composants volatils du résidu de pulvérisation pendant 1 heure 30 à 105 °C.

Le débit en composition aérosol ($D_{CA}$) correspond à la quantité de composition aérosol (jus + propulseur) sortant du dispositif aérosol par unité de temps. Il est exprimé en mg/s et est mesuré par la différence entre le poids de l'aérosol avant ($M_0$) et après ($M_1$) 10 secondes de vaporisation:

$$D_{CA} = (M_0 - M_1) / 10.$$

Les caractéristiques de débit en matière sèche et de pouvoir mouillant des dispositifs aérosols selon l'invention dépendent d'une part de la composition aérosol, et d'autre part au moyen de distribution, les deux devant être appropriés pour obtenir les caractéristiques recherchées. Parmi les paramètres susceptibles d'influencer ces caractéristiques, on citera plus particulièrement la concentration en matière sèche ($C_{MS}$), le débit en composition aérosol ($D_{CA}$) et la phase de la composition aérosol.

D'une manière avantageuse, la concentration en matière sèche ($C_{MS}$) est comprise entre 2,5 et 15 % en poids par rapport au poids total de la composition aérosol (jus + propulseur), de préférence comprise entre 3,5 et 10 % en poids.

Le débit en composition aérosol ($D_{CA}$) sera alors approprié pour obtenir un débit en matière sèche ($D_{MS}$) tel que défini ci-dessus. De manière préférentielle, le $D_{CA}$ sera compris entre 500 et 800 mg/s, plus préférentiellement voisin de 600 mg/s.

La phase de la composition aérosol est de préférence une phase longue, c'est à dire que le rapport pondéral jus/propulseur est supérieur à 1, plus préférentiellement compris entre 1,2 et 3.

De manière avantageuse, le solvant approprié contient au moins 50 % en volume d'alcool, de préférence au moins 70 % en volume d'alcool. Par alcool on entend selon l'invention un alcool aliphatique en $C_1$-$C_4$, de préférence l'éthanol. Il peut s'agir par exemple d'un solvant hydroalcoolique comprenant au moins 50 % en volume d'alcool.

Le propulseur est constitué par les gaz comprimés ou liquéfiés usuellement employés pour la préparation de compositions aérosols. On emploiera de manière préférentielle l'air, le gaz carbonique ou l'azote comprimés, ou encore un gaz soluble ou non dans la composition tel que le diméthyl éther, les hydrocarbures fluorés ou non, et leurs mélanges.

La composition aérosol selon l'invention peut en outre comprendre d'autes polymères fixants, dans la mesure où ils ne viennent pas modifier les caractéristiques du dispositif selon l'invention, en particulier pour ce qui concerne la valeur de Tg du matériau fixant. Ces polymères fixants sont notamment les copolymères d'acide acrylique tels que le terpolymère acide acrylique / acrylate d'éthyle / N-tertiobutylacrylamide commercialisé sous la dénomination ULTRAHOLD STRONG par la société BASF, le terpolymère acide crotonique / acétate de vinyle / t.butyl benzoate de

vinyle, le terpolymère acide méthacrylique / acrylate d'éthyle / acrylate de t.butyle commercialisé sous la dénomination LUVIMER 100P par la société BASF, ou encore les polymères siliconés greffés, à squelette polysiloxanique greffé par au moins un radical hydrocarboné anionique, décrits notamment dans les demandes de brevets EP-A-0 582 152, WO 93/23009 et WO 95/03776, tels que les les polymères siliconés comportant dans leur structure le motif de formule (I) suivant :

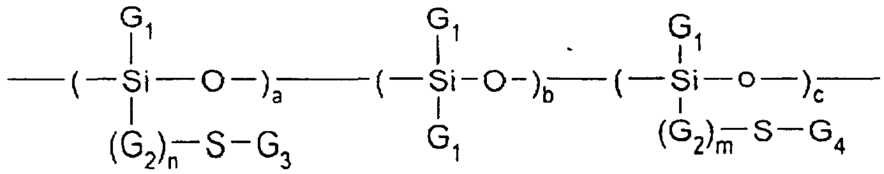

(I)

dans lequel les radicaux $G_1$, identiques ou différents, représentent l'hydrogène ou un radical alkyle en $C_1$-$C_{10}$ ou encore un radical phényle ; les radicaux $G_2$, identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

En fonction de la composition aérosol (jus + propulseur), l'homme du métier saura sélectionner le moyen de distribution approprié pour obtenir les caractéristiques de débit en matière sèche et de pouvoir mouillant recherchées.

Les caractéristiques particulières définies ci-dessus ($C_{MS}$ et phase) peuvent être obtenues en sélectionnant les moyens de distribution appropriés et/ou en agissant sur la formulation.

Les valves appropriées pour les compositions particulières ci-dessus sont des valves droites avec un gicleur de diamètre compris entre 0,35 et 0,60 mm, de préférence compris entre 0,50 et 0,50 mm, avantageusement sans restriction interne ni prise de gaz additionnelle. Il s'agit en particulier des valves commercialisées sous la dénomibnation COSTER T104 RA36/0/4 par la société COSTER, ou de la valve Précision Expérimentale 15130 constituée par un gicleur et un corps de valve de 0,46 mm de diamètres sans prise de gaz additionnelle de la société Précision.

Les diffuseurs appropriés pour les compositions particulières ci-dessus sont en particulier les boutons poussoirs commercialisés sous la dénomination Précision 216903-50AD29 par la société Précision.

La présente invention concerne également un procédé de traitement des fibres kératiniques, caractérisé en ce qu'on applique sur lesdites fibres une composition comprenant un matériau fixant ayant une température de transition vitreuse (Tg) supérieure ou égale à 50 °C, ledit matériau fixant comprenant au moins un polymère à motifs vinyl lactames tel que défini dans les revendications 1 à 5, au moyen d'un dispositif approprié pour obtenir un pouvoir mouillant supérieur ou égal à 50 mg/s et possède une valve droite avec un gicleur de diamètre compris entre 0,35 et 0,60 mm, le matériau fixant étant essentiellement constitué par le polymère à motifs vinyl lactames.

Les exemples ci-après permettent d'illustrer l'invention sans toutefois en limiter la portée. Dans les exemples, « ma » signifie « matière active ».

**Exemple 1 : Importance du « pouvoir mouillant » pour laquer**

On prépare les deux dispositifs aérosol suivants:

Dispositif 1 (selon l'invention)

On prépare le jus suivant:

Composition A

| | |
|---|---|
| Terpolymère vinyl caprolactame / vinyl pyrrolidone / méthacrylate de diméthyl amino éthyle commercialisé sous la dénomination Gaffix VC 713 par la société ISP | 6,0 g ma |
| Monométhyl éther de tripropylène glycol commercialisé sous la dénomination Dowanol TPM par la société Dow Chemical | 0,06 g |

(suite)

| | |
|---|---|
| Silicone commercialisée sous la dénomination DC 190 Fluid par la société Dow Corning | 0,30 g |
| Ethanol qs | 100,00 g |

On introduit 65g de ce jus dans un flacon aérosol que l'on équipe d'une valve Précision Expérimentale 15130, puis on ajoute 35 g de diméthyl éther comme propulseur, et un bouton poussoir Précision 216903-50AD29.
Les caractéristiques de ce dispositif sont les suivantes:

| | |
|---|---|
| Tg du matériau fixant : | 61 °C |
| Pouvoir mouillant | 110 mg/s |

Dispositif 2 (comparatif)

On prépare le jus suivant:

Composition B

| | |
|---|---|
| Terpolymère vinyl caprolactame / vinyl pyrrolidone / méthacrylate de diméthyl amino éthyle commercialisé sous la dénomination Gaffix VC 713 par la société ISP | 9,0 g ma |
| Monométhyl éther de tripropylène glycol commercialisé sous la dénomination Dowanol TPM par la société Dow Chemical | 0,09 g |
| Silicone commercialisée sous la dénomination DC 190 Fluid par la société Dow Corning | 0,45 g |
| Ethanol qs | 100,00 g |

On introduit 37 g de ce jus et 20 g de pentane dans un flacon aérosol que l'on équipe d'une valve Précision P155/S90 (commercialisée par la société Précision), puis on ajoute 43 g de diméthyl éther comme propulseur, et un bouton poussoir Précision 21 6943-50 (commercialisé par la société Précision).
Les caractéristiques de ce dispositif sont les suivantes:

| | |
|---|---|
| Tg du matériau fixant | 61 °C |
| Pouvoir mouillant | 30 mg/s |

On teste ces deux dispositifs sur dix têtes par demi-têtes. Les tests montrent dans tous les cas que seul lecoté traité par le dispositif selon l'invention permet d'obtenir un effet de fixation cosmétique recherché. Par contre, le dispositif 2 (comparatif) ne permet pas d'obtenir un résultat de fixation cosmétique satisfaisant.

**Exemple 2 : Importance de la « Tg » pour obtenir de bonnes propriétés cosmétiques**

On prépare les jus suivants:

Composition C

| | |
|---|---|
| Terpolymère vinyl caprolactame / vinyl pyrrolidone / méthacrylate de diméthyl amino éthyle commercialisé sous la dénomination Gaffix VC 713 par la société ISP | 6,0 g ma |
| Ethanol qs | 100,00 g |

Composition D

| | |
|---|---|
| Terpolymère vinyl caprolactame / vinyl pyrrolidone / méthacrylate de diméthyl amino éthyle commercialisé sous la dénomination Gaffix VC 713 par la société ISP | 6,0 g ma |
| Monométhyl éther de tripropylène glycol commercialisé sous la dénomination Dowanol TPM par la société Dow Chemical | 2,50 g |
| Ethanol qs | 100,00 g |

On prépare les dispositifs aérosols 3 et 4 en introduisant dans des flacons aérosol 65 g des compositions C et D, respectivement. On équipe les deux flacons d'une valve Précision Expérimentale 15130, puis on ajoute 35 g de diméthyl éther comme propulseur, et un bouton poussoir Précision 216903-50AD29.
Pour les deux dispositifs, le pouvoir mouillant de 110 mg/s.
Les Tg des matériaux fixants dans les deux dispositifs sont les suivants:

| | |
|---|---|
| Tg dispositif 3 (selon l'invention) | 61 °C |
| Tg dispositif 4 (comparatif) | 29 °C |

On compare les performances des deux dispositifs en pulvérisant sur 10 têtes les deux compositions par demi-têtes. Dans tous les cas, les pouvoirs fixants sont satisfaisants et comparables. En revanche, le toucher est plus agréable du côté traité par le dispositif 3 selon l'invention par rapport au côté traité par le dispositif 4 comparatif, de même que le démêlage est plus aisé de ce même côté, ainsi que le toucher après démêlage est jugé plus agréable.

**Exemple 3: Dispositifs selon l'invention**

On prépare les jus suivants:

Composition E

| | |
|---|---|
| Vinyl caprolactame | 7,0 g ma |
| Ethanol qs | 100,00 g |

Composition F

| | |
|---|---|
| Terpolymère vinyl caprolactame / vinyl pyrrolidone / méthacrylate de diméthyl amino éthyle commercialisé sous la dénomination Gaffix VC 713 par la société ISP | 4,0 g ma |
| Terpolymère acide acrylique / acrylate d'éthyle / N-tertiobutylacrylamide commercialisé sous la dénomination ULTRAHOLD STRONG par la société BASF | 2,0 g ma |
| Ethanol qs | 100,00 g |

On prépare les dispositifs aérosols 5 et 6 selon l'invention en introduisant dans des flacons aérosol 65 g des compositions E et F, respectivement. On équipe les deux flacons d'une valve Précision Expérimentale 15130, puis on ajoute 35 g de diméthyl éther comme propulseur, et un bouton poussoir Précision 216903-50AD29.
Ces deux dispositifs selon l'invention permettent d'obtenir les résultats de fixation cosmétique, de toucher et de démêlage conformes à l'invention.

**Revendications**

1. Dispositif aérosol constitué par un récipient contenant une composition aérosol constituée d'une part par une phase liquide (ou jus) contenant au moins un matériau fixant dans un solvant approprié et d'autre part un propulseur, et un moyen de distribution de ladite composition aérosol, **caractérisé en ce que** le matériau fixant a une température de transition vitreuse (Tg) supérieure ou égale à 50 °C, ledit matériau fixant comprenant au moins un polymère à motifs vinyl lactames, et **en ce que** le dispositif est approprié pour obtenir un pouvoir mouillant supérieur ou égal à 50 mg/s et possède une valve droite avec un gicleur de diamètre compris entre 0,35 et 0,60 mm, le matériau fixant étant essentiellement constitué par le polymère à motifs vinyl lactames.

2. Dispositif selon la revendication 1, **caractérisé en ce que** le polymère à motifs vinyl lactames comprennent des motifs de formule

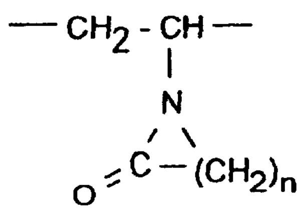

dans laquelle n est indépendamment 3, 4 ou 5.

3. Dispositif selon l'une des revendications 1 à 2, **caractérisé en ce que** les polymères à motifs vinyl lactames sont des copolymères comprenant également des motifs de formule

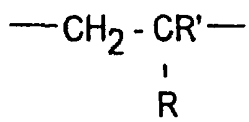

dans laquelle

R représente indépendamment un radical carboxy, un radical carbalcoxy, un radical acyloxy, le groupement alkoxy du radical carbalcoxy pouvant être substitué par au moins un radical hydroxy, amino, alkylamino ou dialkylamino, ou un radical aryle, en particulier phényle, éventuellement substitué par au moins un radical alkyle, et
R' représente indépendamment un atome d'hydrogène ou un radical alkyle.

4. Dispositif selon l'une des revendications 1 à 4, **caractérisé en ce que** les polymères à motifs vinyl lactames sont choisis parmi les polyvinyl pyrrolidones, les polyvinyl caprolactames, les copolymères polyvinyl pyrrolidone /acétate de vinyle, les copolymères polyvinyl pyrrolidone / méthacrylate de diaminoéthyle non quaternisé, les terpolymères polyvinyl pyrrolidone / acétate de vinyle / propionate de vinyle, les terpolymères vinyl caprolactame / vinyl pyrrolidone / méthacrylate de diméthyl amino éthyle et les terpolymères acide (méth)acrylique / (méth)acrylates / vinyl pyrrolidone.

5. Dispositif selon l'une des revendications 1 à 5, **caractérisé en ce qu'**il est approprié pour obtenir un pouvoir mouillant compris entre 50 mg/s et 500 mg/s.

6. Dispositif selon l'une des revendications 1 à 6, **caractérisé en ce qu'**il est approprié pour obtenir un débit en matière sèche supérieur ou égal à 20 mg/s, de préférence compris entre 20 et 60 mg/s.

7. Dispositif selon l'une des revendications 1 à 7, **caractérisé en ce que** la concentration en matière sèche ($C_{MS}$) est comprise entre 2,5 et 15 % en poids par rapport au poids total de la composition aérosol (jus + propulseur), de préférence comprise entre 3,5 et 10 % en poids.

**8.** Dispositif selon l'une des revendications 1 à 8, **caractérisé en ce que** le débit en composition aérosol ($D_{CA}$) est compris entre 500 et 800 mg/s, de préférence voisin de 600 mg/s.

**9.** Dispositif selon l'une des revendications 1 à 9, **caractérisé en ce que** le rapport pondéral jus/propulseur est supérieur à 1, de préférence compris entre 1,2 et 3.

**10.** Dispositif selon l'une des revendications 1 à 10, **caractérisé en ce que** le solvant approprié contient au moins 50 % en volume d'alcool, de préférence au moins 70 % en volume d'alcool.

**11.** Dispositif selon l'une des revendications 1 à 11, **caractérisé en ce qu'**il contient d'autres polymères fixants.

**12.** Procédé de traitement des fibres kératiniques, **caractérisé en ce qu'**on applique sur lesdites fibres une composition comprenant un matériau fixant ayant une température de transition vitreuse (Tg) supérieure ou égale à 50 °C, ledit matériau fixant comprenant au moins un polymère à motifs vinyl lactames tel que défini dans les revendications 1 à 5, au moyen d'un dispositif approprié pour obtenir un pouvoir mouillant supérieur ou égal à 50 mg/s et possède une valve droite avec un gicleur de diamètre compris entre 0,35 et 0,60 mm, le materiau fixant étant essentiellement constitué par le polymère à motifs vinyl lactames.

## Patentansprüche

**1.** Aerosolvorrichtung, die aus einem Behälter, der eine Aerosolzusammensetzung enthält, die aus einer flüssigen Phase (oder Flüssigkeit), die in einem geeigneten Lösungsmittel mindestens einen festigenden Stoff enthält, und aus einem Treibmittel besteht, und einer Einrichtung zur Verteilung der Aerosolzusammensetzung besteht, **dadurch gekennzeichnet, dass** der festigende Stoff eine Glasübergangstemperatur (Tg) von 50 °C oder darüber aufweist, wobei der festigende Stoff mindestens ein Polymer mit Vinyllactam-Einheiten umfasst, und dadurch, dass die Vorrichtung geeignet ist, ein Benetzungsvermögen von 50 mg/s oder darüber zu erzielen, und ein gerades Ventil mit einem Düsendurchmesser im Bereich von 0,35 bis 0,60 mm besitzt, wobei der festigende Stoff im Wesentlichen aus dem Polymer mit Vinyllactam-Einheiten besteht.

**2.** Vorrichtung nach Anspruch 1, **dadurch gekennzeichnet, dass** das Polymer mit Vinyllactam-Einheiten Einheiten der folgenden Formel enthält:

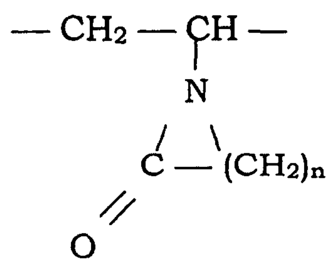

wobei n 3, 4 oder 5 bedeutet.

**3.** Vorrichtung nach einem der Ansprüche 1 oder 2, **dadurch gekennzeichnet, dass** die Polymere mit Vinyllactam-Einheiten Copolymere sind, die ferner Einheiten der folgenden Formel enthalten:

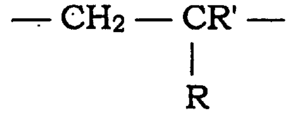

worin bedeuten:

R unabhängig Carboxy, Carbalkoxy, Acyloxy, wobei die Alkoxygruppe der Carbalkoxygruppe mit mindestens

einer Hydroxy-, Amino-, Alkylamino- oder Dialkylaminogruppe substituiert sein kann, oder Aryl, insbesondere Phenyl, wobei die Arylgruppe gegebenenfalls mit mindestens einer Alkylgruppe substituiert sein kann; und

R' Wasserstoff oder Alkyl.

**4.** Vorrichtung nach einem der Ansprüche 1 bis 4, **dadurch gekennzeichnet, dass** die Polymere mit Vinyllactam-Einheiten unter den Polyvinylpyrrolidonen, Polyvinylcaprolactamen, Polyvinylpyrrolidon/Vinylacetat-Copolymeren, nicht quaternisierten Polyvinylpyrrolidon/Diaminoethylmethacrylat-Copolymeren, Polyvinylpyrrolidon/Vinylacetat/Vinylpropionat-Terpolymeren, Vinylcaprolactam/Vinylpyrrolidon/ Dimethylaminoethylmethacrylat-Terpolymeren und (Meth)acrylsäure/(Meth)acrylate/Vinylpyrrolidon-Terpolymeren ausgewählt sind.

**5.** Vorrichtung nach einem der Ansprüche 1 bis 5, **dadurch gekennzeichnet, dass** sie geeignet ist, ein Benetzungsvermögen im Bereich von 50 bis 500 mg/s zu erzielen.

**6.** Vorrichtung nach einem der Ansprüche 1 bis 6, **dadurch gekennzeichnet, dass** sie geeignet ist, einen Durchsatz der Trockensubstanz von mindestens 20 mg/ s und vorzugsweise im Bereich von 20 bis 60 mg/s zu erzielen.

**7.** Vorrichtung nach einem der Ansprüche 1 bis 7, **dadurch gekennzeichnet, dass** die Trockensubstanzkonzentration ($K_{TS}$), bezogen auf das Gesamtgewicht der Aerosolzusammensetzung (Flüssigkeit + Treibmittel), im Bereich von 2,5 bis 15 Gew.-% und vorzugsweise im Bereich von 3,5 bis 10 Gew.-% liegt.

**8.** Vorrichtung nach einem der Ansprüche 1 bis 8, **dadurch gekennzeichnet, dass** der Durchsatz der Aerosolzusammensetzung ($D_{AZ}$) im Bereich von 500 bis 800 mg/ s und noch bevorzugter in der Gegend von 600 mg/ s liegt.

**9.** Vorrichtung nach einem der Ansprüche 1 bis 9, **dadurch gekennzeichnet, dass** das Gewichtsverhältnis Flüssigkeit/Treibmittel größer 1 ist und vorzugsweise im Bereich von 1,2 bis 3 liegt.

**10.** Vorrichtung nach einem der Ansprüche 1 bis 10, **dadurch gekennzeichnet, dass** das geeignete Lösungsmittel mindestens 50 Vol-% Alkohol und vorzugsweise mindestens 70 Vol-% Alkohol enthält.

**11.** Vorrichtung nach einem der Ansprüche 1 bis 11, **dadurch gekennzeichnet, dass** sie weitere festigende Polymere enthält.

**12.** Verfahren zur Behandlung von Keratinfasern, **dadurch gekennzeichnet, dass** eine Zusammensetzung, die einen festigenden Stoff mit einer Glasübergangstemperatur (Tg) von mindestens 50 °C enthält, wobei der festigende Stoff mindestens ein Polymer mit Vinyllactam-Einheiten nach einem der Ansprüche 1 bis 5 umfasst, mit einer

## Claims

**1.** Aerosol device consisting of a container containing an aerosol composition consisting, on the one hand, of a liquid phase (or fluid) containing at least one fixing material in a suitable solvent and, on the other hand, a propellant, and a means for distributing the said aerosol composition, **characterized in that** the fixing material has a glass transition temperature (Tg) of greater than or equal to 50°C, the said fixing material comprising at least one polymer containing vinyllactam units, and **in that** the device is suitable for obtaining a wetting power of greater than or equal to 50 mg/s and has a straight valve with a nozzle of diameter between 0.35 and 0.60 mm, the fixing material consisting essentially of the polymer containing vinyllactam units.

**2.** Device according to Claim 1, **characterized in that** the polymer containing vinyllactam units comprises units of formula

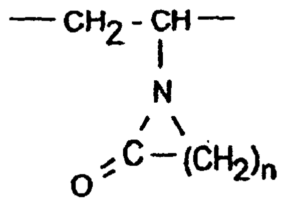

in which n is, independently, 3, 4 or 5.

**3.** Device according to either of Claims 1 and 2, **characterized in that** the polymers containing vinyllactam units are copolymers also comprising units of formula

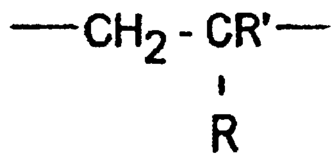

in which

R represents, independently, a carboxyl radical, a carbalkoxy radical, an acyloxy radical, it being possible for the alkoxy group of the carbalkoxy radical to be substituted with at least one hydroxyl, amino, alkylamino or dialkylamino radical, or an aryl radical, in particular phenyl, optionally substituted with at least one alkyl radical, and

R' represents, independently, a hydrogen atom or an alkyl radical.

**4.** Device according to one of Claims 1 to 3, **characterized in that** the polymers containing vinyllactam units are chosen from polyvinylpyrrolidones, polyvinylcaprolactams, polyvinylpyrrolidone/vinyl acetate copolymers, polyvinylpyrrolidone/non-quaternized diaminoethyl methacrylate copolymers, polyvinylpyrrolidone/vinyl acetate/vinyl propionate terpolymers, vinylcaprolactam/vinylpyrrolidone/dimethylaminoethyl methacrylate terpolymers and (meth)acrylic acid/(meth)acrylates/vinylpyrrolidone terpolymers.

**5.** Device according to one of Claims 1 to 4, **characterized in that** it is suitable for obtaining a wetting power of between 50 mg/s and 500 mg/s.

**6.** Device according to one of Claims 1 to 5, **characterized in that** it is suitable for obtaining a flow rate of solids of greater than or equal to 20 mg/s, preferably between 20 and 60 mg/s.

**7.** Device according to one of Claims 1 to 6, **characterized in that** the solids concentration ($C_{SM}$) is between 2.5 and 15% by weight relative to the total weight of the aerosol composition (fluid + propellant), preferably between 3.5 and 10% by weight.

**8.** Device according to one of Claims 1 to 7, **characterized in that** the flow rate of aerosol composition ($D_{AC}$) is between 500 and 800 mg/s, preferably close to 600 mg/s.

**9.** Device according to one of Claims 1 to 8, **characterized in that** the fluid/propellant weight ratio is greater than 1, preferably between 1.2 and 3.

**10.** Device according to one of Claims 1 to 9, **characterized in that** the appropriate solvent contains at least 50% by volume of alcohol, preferably at least 70% by volume of alcohol.

**11.** Device according to one of Claims 1 to 10, **characterized in that** it contains other fixing polymers.

**12.** Process for treating keratin fibres **characterized in that** a composition comprising a fixing material having a glass transition temperature (Tg) of greater than or equal to 50°C, the said fixing material comprising at least one polymer containing vinyllactam units as defined in Claims 1 to 4, is applied to the said fibres by means of a suitable device in order to obtain a wetting power of greater than or equal to 50 mg/s and has a straight valve with a nozzle of diameter between 0.35 and 0.60 mm, the fixing material consisting essentially of the polymer containing vinyllactam units.